# EUROPEAN PATENT SPECIFICATION

(11) **EP 1 001 034 B1**
(45) Date of publication and mention of the grant of the patent: **05.10.2005**
(21) Application number: 99204324.0
(22) Date of filing: 22.01.1992
(51) Int. Cl.: C12P 7/64, A23D 9/00, A23C 11/04, A61K 31/20, A61K 7/06, A61K 7/48, A23L 1/30

(54) **Arachidonic acid and methods for the production and use thereof**
Arachidonsäure und Verfahren zu ihrer Herstellung sowie Verwendung derselben
Acide arachidonique, ses procédés de production et d'utilisation

(30) Priority: 24.01.1991 US 645454
(43) Date of publication of application: 17.05.2000
(62) Divisional of application: 92904428.7
(73) Proprietor: MARTEK CORPORATION, Columbia, MD 21045 (US)
(72) Inventor: Kyle, David, Catonsville, MD 21228 (US)
(74) Representative: Cockerton, Bruce Roger

(56) References cited:
- EP-A- 0 140 805
- EP-A- 0 148 303
- H. YAMADA ET AL.: "Biotechnological processes for production of poly-unsaturated fatty acids" JOURNAL OF DISPERSION SCIENCE AND TECHNOLOGY, vol. 10, no. 4&5, 1989, pages 561-579, XP000884958
- DATABASE WPI Section Ch, Week 9004 Derwent Publications Ltd., London, GB; Class B05, AN 90-026632 XP002131309 "Prepn. of oil and fat contg. highly unsatd. fatty acid - comprises culturing specific microbial strain which can produce arachidonic acid with oil and fat as carbon source" & JP 01 304892 A (SUNTORY LTD), 8 December 1989 (1989-12-08)
- PATENT ABSTRACTS OF JAPAN vol. 13, no. 227 (C-600) [3575], 25 May 1989 (1989-05-25) & JP 01 038007 A (LION CORP), 8 February 1989 (1989-02-08)

## Description

This invention relates to the production of arachidonic acid, to compositions containing arachidonic acid and to uses thereof.

Arachidonic acid (ARA) is a long chain polyunsaturated fatty acid (PUFA) of the omega-6 class (5,8,11,14-eicosatetraenoic acid, i.e., 20:4). ARA is the most abundant C₂₀ PUFA in the human body. It is particularly prevalent in organ, muscle and blood tissues, serving a major role as a structural lipid associated predominantly with phospholipids in blood, liver, muscle and other major organ systems. In addition to its primary role as a structural lipid, ARA also is the direct precursor for a number of circulating eicosenoids such as prostaglandin E₂ (PGE₂), prostacyclin I₂ (PGI₂), thromboxane A₂ (TₓA₂), and leukotirenes B₄ (LTB₄) and C₄ (LTC₄). These eicosenoids exhibit regulatory effects on lipoprotein metabolism, blood rheology, vascular tone, leucocyte function and platelet activation.

Despite its importance to human metabolism, ARA cannot be synthesized in humans de novo. ARA is synthesized by the elongation and desaturation of linoleic acid (LOA), an essential fatty acid. This process requires the presence of the enzyme Δ6-desaturase, an enzyme present in the human body in low levels, Burre et al., Lipids, 25:354-356 (1990). Accordingly, most ARA must be provided in the diet, and this is especially important during times of very rapid body growth, such as infancy.

During the first year of its life, an infant can double or triple its weight. Consequently, elevated levels of dietary ARA are required. To satisfy this increased demand, human breast milk contains high levels of ARA. Sanders et al., Am. J. Clin. Nutr., 31:805-813 (1978). ARA is the most prevalent C₂₀ PUFA in breast milk. Of those mothers, especially vegetarians, who do breast-feed their infants, many would benefit from additional dietary ARA. However, many mothers do not breast feed their infants, or do not breast feed for the entire period of rapid infant growth, choosing instead to utilize an infant formula.

No commercial infant formulas known to Applicant contain ARA. U.S. Patent No. 4,670,285 (Clandinin et al.), incorporated herein by reference, discloses the infant's requirement for fatty acids including ARA. To provide these fatty acids, Clandinin et al. suggest a blend of egg yolk, fish oil or red blood cell phospholipids and vegetable oils as the fat component of a proposed infant formula. However, fish oil contains high quantities of eicosapentaneoic acid (EPA). EPA is known to depress ARA synthesis in infants. Carlson, et al., INFORM, 1:306 (1990).Thus, it would be desirable to be able to provide ARA without also providing additional EPA. Furthermore, egg yolks contain a relatively low concentration of ARA, such that Clandinin et al.'s mixture is not economically viable.

Because ARA is present in animal, but not vegetable, oils, its production in commercial quantities has remained a desirable, but elusive, goal. Shinmen, et al., Microbiol. Biotech. 31:11-16 (1989), have reported the production of ARA by an isolated fungus, *Mortierella alpina*, using conventional stirred tank fermentation. (See also Japanese Patent 1,215,245 to Shinmen et al.). After culturing, the organisms are harvested, dried and their lipids extracted from the fungal biomass with an organic solvent and the lipids chemically (covalently) modified. For example, the lipid mixture is hydrolyzed or converted to ethyl esters and then combined with cyclodextrin prior to use as a dietary supplement. Shinmen et al. do not disclose or suggest the administration of unmodified microbial oils.

*Porphyridium cruentum*, a red microalgae, can be grown in ponds in large quantities and has a lipid content which can contain up to 40% ARA. Ahern, et al. Biotech. Bioeng. 25:1057-1070 (1983). Unfortunately, the ARA is primarily associated with galactolipids, a complex polar lipid not present in breast milk. Thus, not only is the total usable ARA produced a fraction of one percent of the biomass, but the form of the ARA is not suitable for use as an additive to infant formula without further modification.

U.S. Patent No. 4,870,011 (Suzuki et al.) discloses a method for obtaining lipids such as γ-linolenic acid from fungi of the genus Mortierella. The γ-linolenic acid is purified from the mixture of lipids contained in the fungi.

DE 3603000A1 (Milupa) discloses a highly polyunsaturated acid fat mixture and its use as the fat component of an infant formula. The fat mixture has a high content of ARA and docosahexanoic (DHA) acids in a ratio of 2.5:1 respectively, as well as a high content of cholesterol. Sources of the fatty acids are listed as being certain types of macroalgae, fish oils, organ fats from beef and pork or highly refined egg yolk oil. A source of the DHA and ARA is said to be macroalgae of the phaecophyte and rhodophyte types. There is no suggestion to use any microbes as a source of oil. Algal and fish oils also typically include EPA which depresses ARA synthesis in vivo. Additionally, highly refined egg yolk oil is not an economical source of ARA. Moreover, there is no disclosure therein of an ARA-concentrated additive for supplementing pre-existing infant formula.

H. Yamada et al. in Journal of Dispersion Science and Technology, vol. 10 (4 and 5), pages 561-579 (1989), describe biotechnological processes for the production of polyunsaturated fatty acids. These methods consist of culturing fungal mycelia such as *Mortierella alpina*, and extracting an oil rich in polyunsaturated fatty acids from the mycelium. Among the fatty acids that can be produced in high yield is arachidonic acid (ARA).

JP-A-01 304 892 describes the preparation of lipids containing ARA by culturing a nucrobial strain, such as *Mortierella* or *Pythium* in a culture medium containing oil and fat as the carbon source. The unsaturated fatty acids produced by the culture depend on the oil and fat used as the carbon source.

JP-A-01 038 007 describes a pharmaceutical preparation for topical application to the skin, containing an ARA-containing lipid as the active ingredient.

EP-A-0 140 805 describes an infant formula containing long chain polyunsaturated fatty acids selected from ARA, linolenic acid and docosahexaenoic acid (DHA) contained in a lipid extract of placenta.

EP-A-0 148 303 describes a dietetic composition containing phospholipids, including ARA phospholipids obtained from vegetable sources such as linseed oil.

Accordingly, there remains a need for an economical, commercially feasible method of producing ARA, preferably without concomitant production of EPA. It is an object of the present invention to satisfy that need.

It is a further object of the invention to provide an additive, and a source for that additive, for use in an infant formula such that the ARA levels in the formula approximate those levels in human breast milk.

It is an additional object of this invention to provide an ARA-containing fungal oil for use in enteral, parenteral or dermal products.

### Summary of the Invention

This invention relates to the production and use of arachidonic acid containing fungal oil (ARASCO) and to compositions containing such oils. The oil can be referred to as a single cell oil. Fungi are cultivated under oil-producing conditions, harvested and the oil extracted and recovered. The oil, without further chemical modification, can be used directly to provide supplemental ARA to persons requiring such, including newborn infants, pregnant or nursing women or persons exhibiting ARA-deficient pathologies. Advantages of the invention include its ease of production, and high purity, and lack of detectable amounts of EPA.

### Detailed Description of the Preferred Embodiment of the Invention

The present invention succeeds in providing an economical source of arachidonic acid (ARA). In one embodiment, this invention relates to a method for the production of an arachidonic acid-containing fungal oil (ARASCO) which is substantially free of eicosapentaneoic acid (EPA). As used herein, "substantially free" means that the EPA is present in less than about one fifth of the amount of ARA in the oil. This oil, a single cell oil, can be administered directly, in an unmodified form. As used herein "unmodified" means that the chemical properties of the fatty acids, or the oils themselves, have not been covalently altered. Thus, for example, a temporary modification to the ARASCO or ARA which could be reversed following uptake of the oil would not be beyond the scope of this invention.

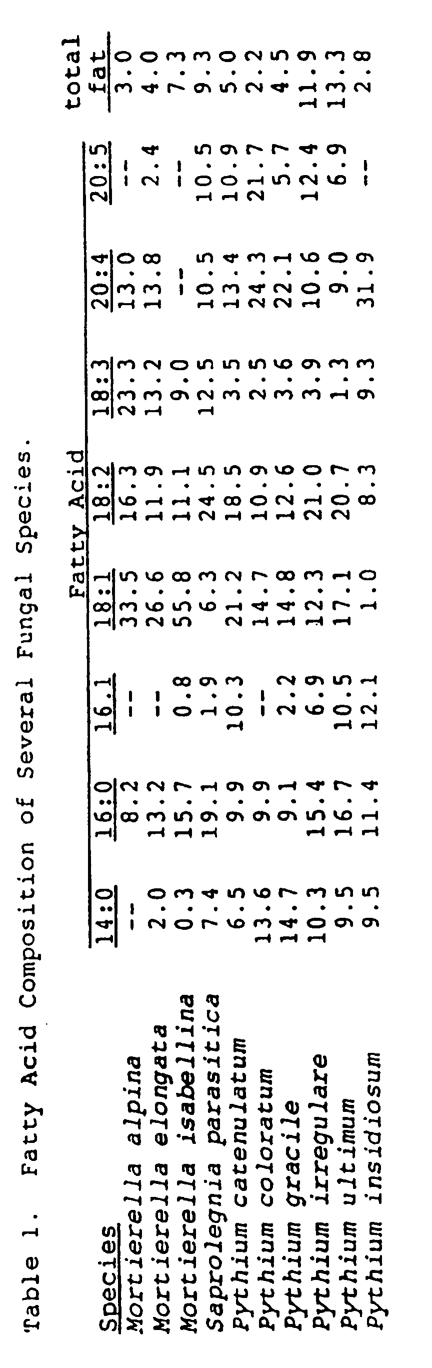

Of those fungal species which previously have had their fatty acids characterized, it has been found that most do not make ARA. Weete, J.D., Fungal Lipid Biochemistry, Plenum Press, N.Y. (1974). Of those species which do make ARA, many, including all previously characterized *Pythium* species, produce significant quantities of eicosapentaenoic acid (EPA) in addition to ARA. Table 1 sets forth the fatty acid profile of *P. insidiosum* as well as the fatty acid profile of other species of fungi. Unexpectedly, it has been found that *P. insidiosum* produces ARA without concomitant production of EPA. As with fish oils, high EPA levels in dietary supplements result in a depression of the ability to form ARA from dietary linoleic acid (LOA). Accordingly, while those fungal species producing both ARA and EPA can be utilized in the process of this invention, it is preferable to use species which do not produce significant quantities of EPA. Such preferred species include *Pythium insidiosum* and *Mortierella alpina*. Both species are available commercially and are on deposit with the American Type Culture Collective in Rockville, Maryland, having accession numbers 28251 and 42430, respectively. Throughout this disclosure, unless otherwise expressly stated, *P. insidiosum* will be the representative fungal species.

One of the significant problems which an embodiment of the present invention overcomes, is the depression of ARA biosynthesis in infants caused by the presence of enhanced dietary levels of EPA. This problem can be corrected by providing ARA for use in infant formula at levels substantially similar to those found in human breast milk. Typically in human breast milk, the ratio of ARA:EPA is about 20:1 respectively. The present invention specifically contemplates any microbial oil which provides a sufficient amount of ARA to overcome the negative effects of dietary EPA. Preferably, the use of the ARA-containing oil will result in an ARA:EPA ratio of at least about 5:1. More preferably, the ratio will be at least about 10:1 and, most preferably, it will be at least about 20:1. As can be seen, the higher the amount of ARA in the end product, with respect to the amount of EPA, the more desirable is the result.

In a process of the present invention, the fungi are cultivated under suitable ARA-containing oil producing cultivating conditions. In general, techniques of fungal cultivation are well known to those of skill in the art and those techniques can be applied to the present inventive process. For example, cultivation of an inoculating amount of fungus can occur in submerged culture in shake flasks. The flask is provided with a growth medium, seeded with fungal mycelium, and grown on a reciprocating shaker for about three to four days.

The composition of the growth medium can vary but always contains carbon and nitrogen sources. A preferred carbon source is glucose, amounts of which can range from about 10-100 grams glucose per liter of growth medium. Typically about 15 grams/liter are utilized for shaker flask culture. The amount can be varied depending upon the desired density of the final culture. Other carbon sources which can be used include molasses, high fructose corn syrup, hydrolyzed starch or any other low cost conventional carbon source used in fermentation processes. Additionally, lactose can be provided as a carbon source for *P. insidiosum*. Thus, whey permeate, which is high in lactose and is a very low cost carbon source, can be used as a substrate. Suitable amounts of these carbon sources can readily be determined by those of skill in the art. Usually, additional carbon needs to be added during the course of the cultivation. This is because the organisms use so much carbon that adding it all in a batch mode could prove unwieldy.

Nitrogen typically is provided in the form of yeast extract at a concentration of from about 2 to about 15 grams extract per liter of growth medium. Preferably, about four grams per liter are provided. Other nitrogen sources can be used, including peptone, tryptone, cornsteep liquor, etc. The amount to be added of these sources can easily be determined by those of skill in the art. Nitrogen can be added in a batch mode, i.e. all at one time prior to cultivation.

After cultivation for 3-4 days at a suitable temperature, typically about 25-30°C, an amount of fungi has grown which is sufficient for use as an inoculum in a conventional stirred tank fermentor (STF). Such fermentors are known to those of skill in the art and are commercially available. Fermentation can be carried out in batch, fed-batch, or continuous fermentation modes. Preferably, the STF is equipped with a Rushton-type turbine impeller.

The fermentor is prepared by adding the desired carbon and nitrogen sources. For example, a 1.5 liter fermentor can be prepared by mixing about 50 grams of glucose and about 15 grams of yeast extract per liter of tap water. As previously discussed, other carbon or nitrogen sources or mixtures thereof can be used.

The reactor containing the nutrient solution should be sterilized by, for example, heating prior to inoculation. After cooling to about 30°C, the inoculum can be added, and cultivation initiated. Gas exchange is provided by air sparging. The air sparging rate can vary, but preferably is adjusted to from about 0.5 to about 4.0 VVM (volume of air per volume of fermentor per minute). Preferably the dissolved oxygen level is kept at from about 10% to about 50% of the air saturation value of the solution. Accordingly, adjustments in the sparge rate may be required during cultivation. Agitation is desirable. The agitation is provided by the impeller. Agitation tip speed preferably is set within the range of from about 50 cm/sec to about 500 cm/sec, preferably from about 100 to 200 cm/sec.

In general, the amount of inoculum can vary. Typically, from about 2% to about 10% by volume of inoculum can be used. Preferably, in a fermentor seed train about 5% by volume of inoculum can be used.

Nutrient levels should be monitored. When glucose levels drop below 5 g/l, additional glucose should be added. A typical cultivation cycle utilizes about 100 grams of glucose and about 15 grams of yeast extract per liter. It is desirable to deplete the nitrogen during the course of the cultivation as this enhances oil production by the fungi. This is especially true when *M. alpina* is used as the production organism.

Occasionally, the culture will produce an excessive quantity of foam. Optionally, an antifoaming agent, such as those known to those of skill in the art, e.g. Mazu 310®, can be added to prevent foam.

The temperature of cultivation can vary. However, those fungi which produce both ARA and EPA tend to produce less EPA and more ARA when cultivated at higher temperatures. For example, when *Mortierella alpina* is cultivated at less than 18°C, it begins to produce EPA. Thus, it is preferable to maintain the temperature at a level which induces the preferential production of ARA. Suitable temperatures are typically from about 25°C to about 30°C.

Preferably, cultivation continues until a desired biomass density is achieved. A desirable biomass is about 25 g/l of the organism. Such a biomass typically is attained within 48-72 hours after inoculation. At this time, the organisms typically contain about 5-40% complex lipids, i.e. oil, of which about 10-40% is ARA, and can be harvested.

Harvesting can be done by any suitable method such as, for example, filtration, centrifugation, or spray drying. Because of lower cost, filtration may be preferred.

After harvesting, the mycelial cake can be extracted. The mycelial cake refers to the collection of biomass resulting after harvest. The cake can be loose or pressed, crumbled or uncrumbled. Optionally, the cake can have any residual water removed, as by vacuum drying or lyophilization, prior to extraction. If this option is selected, it is preferable to use nonpolar solvents to extract the ARA-containing oil. While any non-polar extract is suitable, hexane is preferred.

Alternatively, the wet cake (which typically contains about 30-50% solids) can be crumbled and extracted directly using polar solvents such as ethanol or isopropyl alcohol, or supercritical fluid extraction with solvents such as CO₂ or NO. Preferably, the cakes are crumbled prior to extraction. Advantageously, the present invention permits the economical use of supercritical fluid extraction techniques. McHugh, et al., Supercritical Fluid Extraction, Butterworth (1986). Such techniques are known to those of skill in the art and include those presently applied, for example, to decaffeinate coffee beans. While the yields from both wet and dry extractions are similar, wet extraction generally is a more economical process.

A preferable method of aqueous extraction involves mixing the mycelial biomass with the polar solvent isopropyl alcohol in a suitable reaction kettle. Such kettles are known. The use of three to six parts of solvent per part of biomass is desired. Most preferably, the mixing is done under nitrogen or in the presence of antioxidants to prevent the oxidation of the ARA in the lipid extract. As used herein "lipid extract", "oil", "lipid complex" and "fungal oil" are used interchangeably.

After extracting, the mixture can be filtered to remove the biomass from the solvent containing the lipid extract. At this point, the biomass can be recovered and used as a food supplement. As used herein, "food supplement" means feed or an additive to be mixed with typical feed, such as grain, etc., that can be provided to animals.

The solvent is separated from the lipid extract and also can be recovered for reuse, as by evaporation into a suitable collector, leaving what is referred to herein as the "crude oil." Use of isopropyl alcohol as the solvent desirably results in the removal of any residual water from the crude oil, as the evaporation removes the water/isopropyl alcohol azeotrope which has spontaneously formed.

While the crude oil can be used without further treatment, it also can be further purified. Processes such as those used in the preparation of lecithin from vegetable products, and known to those of skill in the art, can be used in this additional purification step. Such processes do not chemically or covalently modify the ARA-containing lipids or the ARA itself.

Yields vary, but typically are about 5 grams of ARA-containing phospholipid per 100 grams of dried mycelia. In the case of *M. alpina*, an additional 10-30 grams of triglyceride per 100 grams of dry mycelia can be obtained. Either the crude oil or the refined product can be used for administration to humans. Both shall be included within the definition of ARASCO as used herein.

A most preferred object of the invention is to provide an additive for use with human infant formulas, such that the concentration of ARA in such formula closely approximates the concentration of ARA in human breast milk. Table 2 compares the composition of the fatty acids in ARASCO with those in breast milk and infant formula lacking and containing ARASCO.

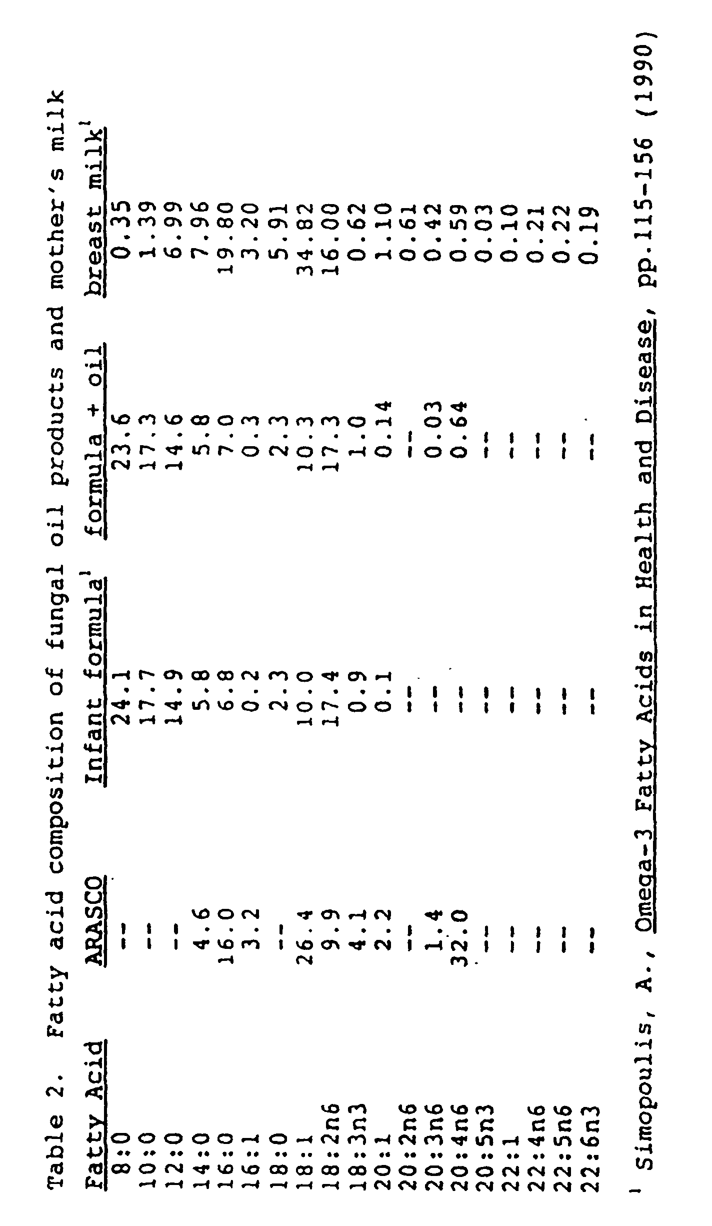

As can be seen, the amount of ARA present in the infant formula supplemented by ARASCO closely approximates the ARA levels in human breast milk. Additionally, the total fatty acid composition of the infant formula has not been significantly altered by the addition of the ARASCO. Typically, between about 50 to about 1000 mg of ARASCO per liter of infant formula can be used. The specific amount of ARASCO required depends upon the ARA content. This can vary from about 10 to about 50% of the fatty acids in the oil. However, typically the ARA content is about 30%. When the ARA content is about 30%, an especially preferred supplementation rate is about 600 to 700 mg of ARASCO per liter of infant formula. Such a rate dilutes the pre-existing fat components of an infant formula such as Similac® (Ross Laboratories, Columbus, Ohio) by only one part ARASCO to fifty parts formula oils. Preferably, the ARASCO is substantially free of EPA.

When *Pythium insidiosum* is used in the described process, the extracted ARA-containing oil is predominantly phospholipid. When *Mortierella alpina* is used in this process, the ARA-containing oil is predominantly triglyceride. Both forms of ARASCO are useful as additives to infant formula. The former not only provides the formula with ARA, but also with an emulsifier, i.e., phosphatidyl choline, which is commonly added to commercial formulas. The oil from *M. alpina* is likely to be more economical to produce.

The ARA-containing oil of the present invention has many uses in addition to its use as an additive for infant formula. As known to those of skill in the art, there are many pathologies associated with ARA deficiencies, such as marasmus (Vajreswari, et al., Metabolism **39**:779-782 (1990)) or atopic diseases (Melnik, B., Monatsschr. Kinderheilta, **138**:162-166 (1990)). In one embodiment of the present invention, those pathologies are treated by administering a pharmaceutically effective amount of the oil of the present invention. The oil can be administered enterally, topically or parenterally, as selected by the provider of health care.

Encapsulation, as known by those of skill in the art, is an effective method of enteral administration. Capsules containing the fungal oil can be administered to those persons requiring or desiring dietary supplementation of ARA. Such a method is particularly effective for administering ARA to pregnant or nursing women.

In instances where ARASCO is being administered to combat ARA deficiency associated pathologies, a pharmaceutically effective amount should be administered. This amount can be determined by those of skill in the art without undue experimentation.

Another embodiment of the present invention entails cosmetic compositions containing ARASCO. Cosmetic compositions refer to those compounds applied as cosmetics. A preferred example of such a composition is a wrinkle cream. Such cosmetic compositions provide an effective means of topically applying ARA to skin to assist in maintaining skin tone.

The invention having been generally described, the following specific non-limiting examples are set forth to further illustrate the invention.

### Example 1. Preparation of P. insidiosum lipid and addition to infant formula

In an 80 liter (gross volume) fermentor, 51 liters of tap water, 1.2 kg glucose, 240 grams of yeast extract and 15 ml of MAZU 210S® antifoam were combined. The fermentor was sterilized at 121°C for 45 minutes. An additional 5 liters of condensate water were added during the sterilization process. The pH was adjusted to 6.2, and approximately 1 liter of inoculum (at a cell density of 5-10g/l) of Pythium insidiosum (ATCC #28251) then was added. The agitation rate was adjusted to 125 RPM (250 cm/sec tip speed) and the aeration rate was set at 1 SCMF (standard cubic feet per minute). At hour 24 in the operation the aeration rate was increased to 3 SCFM. At hour 28 an additional 2 liters of 50% glucose syrup (1 kg glucose) were added. At hour 50 the fermentor was harvested, resulting in a yield of about 2.2 kg wet weight (approximately 15 g dry weight) per liter. Harvested biomass was squeezed to a high solids cake (50% solids) on a suction filter before freeze drying. The dried biomass was ground with a mortar and pestle and extracted with 1 liter of hexane per 200 grams of dry biomass at room temperature under continuous stirring for 2 hours. The mixture then was filtered and the filtrate evaporated to yield about 5-6 grams of crude oil per 100 grams of dry biomass. The biomass then was reextracted with 1 liter of ethanol per 20 grams of dry biomass for 1 hour at room temperature, filtered, and the solvent evaporated yielding an additional 22 grams of crude oil per 100 grams of dry biomass. The second fraction was predominantly phospholipids whereas the first fraction contained a mixture of phospholipids and triglycerides. The combined fractions produced an oil containing about 30-35% arachidonic acid and no detectable EPA. This oil was added dropwise to the commercial infant formula product Simulac® (Ross Laboratories, Columbus, Ohio) at a supplementation rate of 600 mg per liter of prepared medium.

### Example 2. Preparation of M. alpina lipid and addition to infant formula

*Mortierella alpina* (ATCC #42430) was grown in a 2 liter shake flask containing 1 liter of tap water and 20 grams of potato dextrose medium. The flask was under constant orbital agitation and was maintained at 25°C for seven days. After harvesting by centrifugation, the biomass was freeze dried yielding about 8 grams of lipid-rich mycelia. The mycelia was extracted using hexane as in example #1 and about 2.4g of crude oil resulted. This oil contains about 23% arachidonic acid and was added to the commercial formula Similac® dropwise in concentrations of 1000 mg per liter.

## Claims

1. A human nutritive or pharmaceutical composition, containing an unmodified fungal oil comprising fatty acids, wherein said fatty acids comprise from 10 to 50% arachidonic acid (ARA) and no more than one fifth as much eicosapentaenoic acid (EPA) as arachidonic acid (ARA), and further wherein said fungal oil is obtained from *Pythium insidiosum.*

2. The composition of claim 1, wherein the fungal oil containing ARA has been refined prior to incorporation into said composition.

3. The composition of claim 1 or 2, wherein the ratio of ARA to EPA in said fungal oil is greater than 10:1.

4. The composition of any preceding claim, wherein the ratio of ARA to EPA in said fungal oil is greater than 20:1.

5. The composition of any preceding claim, wherein said fungal oil fatty acids comprise 30% to 50% ARA.

6. The composition of any preceding claim, which is an infant formula, and which comprises ARA in an amount closely approximating the amount in human breast milk.

7. The composition of any preceding claim, which is suitable for enteral administration to a human.

8. The composition of any preceding claim, which is suitable for parenteral administration to a human.

9. A method for the production of an arachidonic acid (ARA) containing oil, said oil further comprising no more than one fifth as much eicosapentaenoic acid (EPA) as arachidonic acid (ARA), comprising:
(a) cultivating *Pythium insidiosum* in culture medium containing a carbon source and a nitrogen source, with air sparging and agitation of the culture medium, to induce said *Pythium insidiosum* to produce an oil containing arachidonic acid (ARA) and no more than one fifth as much eicosapentaenoic acid (EPA) as arachidonic acid (ARA);
(b) harvesting said *Pythium insidiosum;*
(c) extracting said oil from said harvested *Pythium insidiosum*; and
(d) recovering said oil.

10. The method of claim 9, wherein the fatty acids present in said oil comprise from 10% to 50% ARA.

11. The method of claim 10, wherein the fatty acids present in said oil comprise from 30% to 50% ARA.

12. The method of any of claims 9 to 11, wherein said oil comprises at least 10 parts ARA per part EPA.

13. The method of claim 12, wherein said oil comprises at least 20 parts ARA per part EPA.

14. The method of any of claims 9 to 13, wherein the dissolved oxygen level in the culture medium is maintained at 10% to 50% of the air saturation value of the medium throughout the cultivation.

15. The method of any of claims 9 to 14, wherein the carbon source is glucose provided at a concentration of from 10 to 100 g glucose per liter of medium and the nitrogen source is yeast extract provided at a concentration of from 2 to 15 g yeast extract per liter of medium.

16. A refined ARA containing fungal oil, wherein the fatty acids present in said fungal oil comprise from 30 to 50% arachidonic acid (ARA) and no more than one fifth as much eicosapentaenoic acid (EPA) as arachidonic acid (ARA), and wherein the fungal oil is selected from the group consisting of fungal oils produced by *Pythium insidiosum,* said fungal oil being suitable for use in the preparation of a composition as claimed in any of claims 2 to 8.

## Patentansprüche

1. Humane Ernährungs- oder pharmazeutische Zusammensetzung, enthaltend ein nicht-modifiziertes, Fettsäuren umfassendes Pilzöl, wobei die Fettsäuren zwischen 10 bis 50% Arachidonsäure (ARA) und nicht mehr als ein Fünftel so viel Eicosapentaensäure (EPA) wie Arachidonsäure (ARA) umfaßt, und wobei ferner das Pilzöl von *Pythium insidiosum* erhalten worden ist.

2. Zusammensetzung nach Anspruch 1, wobei das ARA-enthaltende Pilzöl vor dem Einbringen in die Zusammensetzung verfeinert wird.

3. Zusammensetzung nach Anspruch 1 oder 2, wobei das Verhältnis von ARA zu EPA in dem Pilzöl größer als 10:1 ist.

4. Zusammensetzung nach einem vorhergehenden Anspruch, wobei das Verhältnis von ARA zu EPA in dem Pilzöl größer als 20:1 ist.

5. Zusammensetzung nach einem vorhergehenden Anspruch, wobei die Fettsäuren des Pilzöls 30% bis 50% ARA umfassen.

6. Zusammensetzung nach einem vorhergehenden Anspruch, die eine Kleinkind-Formulierung ist und die ARA in einer Menge, die sich der Menge in humaner Brustmilch annähert, umfaßt.

7. Zusammensetzung nach einem vorhergehenden Anspruch, die für eine enterale Verabreichung an einen Menschen geeignet ist.

8. Zusammensetzung nach einem der vorhergehenden Ansprüche, die für eine parenterale Verabreichung an einen Menschen geeignet ist.

9. Verfahren zur Herstellung eines Arachidonsäure (ARA)-enthaltenden Öls, wobei das Öl ferner nicht mehr als ein Fünftel so viel Eicosapentaensäure (EPA) wie Arachidonsäure (ARA) umfaßt, umfassend
(a) das Kultivieren von *Pythium insidiosum* in einem eine Kohlenstoffquelle und eine Stickstoffquelle enthaltenden Medium unter Hindurchperlen von Luft und Rühren des Kulturmediums, um *Pythium insidiosum* zu veranlassen, ein Öl herzustellen, das Arachidonsäure (ARA) und nicht mehr als ein Fünftel so viel Eicosapentaensäure (EPA) wie Arachidonsäure (ARA) enthält,
(b) Ernten des *Pythium insidiosum,*
(c) Extrahieren des Öls aus dem geernteten *Pythium insidiosum,* und
(d) Zurückgewinnen des Öls.

10. Verfahren nach Anspruch 9, wobei die in dem Öl vorhandenen Fettsäuren 10% bis 50% ARA umfassen.

11. Verfahren nach Anspruch 10, wobei die in dem Öl vorhandenen Fettsäuren 30% bis 50% ARA umfassen.

12. Verfahren nach einem der Ansprüche 9 bis 11, wobei das Öl mindestens 10 Teile ARA pro Teil EPA umfaßt.

13. Verfahren nach Anspruch 12, wobei das Öl mindestens 20 Teile ARA pro Teil EPA umfaßt.

14. Verfahren nach einem der Ansprüche 9 bis 13, wobei während der Kultivierung der Pegel an gelöstem Sauerstoff im Kulturmedium bei 10% bis 50% des Luftsättigungswerts des Mediums beibehalten wird.

15. Verfahren nach einem der Ansprüche 9 bis 14, wobei die Kohlenstoffquelle Glucose, bereitgestellt in einer Konzentration von 10 bis 100 g Glucose pro Liter Medium, ist, und die Stickstoffquelle ein Hefeextrakt, bereitgestellt in einer Konzentration von 2 bis 15 g Hefeextrakt pro Liter Medium, ist.

16. Verfeinertes ARA-enthaltendes Pilzöl, wobei die in dem Pilzöl vorhandenen Fettsäuren 30 bis 50% Arachidonsäure (ARA) und nicht mehr als ein Fünftel so viel Eicosapentaensäure (EPA) wie Arachidonsäure (ARA) umfassen, und wobei das Pilzöl ausgewählt ist aus der Gruppe, bestehend aus Pilzölen, hergestellt durch *Pythium insidiosum,* wobei das Pilzöl zur Verwendung in der Herstellung einer wie in einem der Ansprüche 2 bis 8 definierten Zusammensetzung geeignet ist.

## Revendications

1. Composition nutritive ou pharmaceutique pour l'homme, contenant une huile fongique non modifiée comprenant des acides gras, les acides gras comprenant de 10 à 50 % d'acide arachidonique (ARA) et une quantité d'acide eicosapentaènoïque (EPA) ne représentant pas plus du cinquième de celle de l'acide arachidonique (ARA) et dans laquelle, en outre, l'huile fongique est obtenue à partir de *Pythium insidiosum.*

2. Composition suivant la revendication 1, dans laquelle l'huile fongique contenant ARA a été raffinée avant incorporation dans la composition.

3. Composition suivant la revendication 1 ou 2, dans laquelle le rapport de ARA à EPA de l'huile fongique est supérieur à 10:1.

4. Composition suivant l'une quelconque des revendications précédentes, dans laquelle le rapport de ARA à EPA de l'huile fongique est supérieur à 20:1.

5. Composition suivant l'une quelconque des revendications précédentes, dans laquelle les acides gras de l'huile fongique comprennent de 30 % à 50 % d'ARA.

6. Composition suivant l'une quelconque des revendications précédentes, qui est une formule pour le nourrisson et qui comprend de l'ARA en une quantité très proche de la quantité contenue dans le lait du sein humain.

7. Composition suivant l'une quelconque des revendications précédentes, qui est appropriée à l'administration entérale chez l'homme.

8. Composition suivant l'une quelconque des revendications précédentes, qui est appropriée à l'administration parentérale chez l'homme.

9. Procédé de production d'une huile contenant de l'acide arachidonique (ARA), l'huile contenant, en outre, une quantité d'acide eicosapentaènoïque (EPA) ne représentant pas plus du cinquième de celle de l'acide arachidonique (ARA), dans lequel
a) on cultive *Pythium insidiosum* dans un milieu de culture contenant une source de carbone et une source d'azote avec projection d'air et agitation du milieu de culture pour faire que *Pythium insidiosum* produise une huile contenant de l'acide arachidonique (ARA) et une quantité d'acide eicosapentaènoïque (EPA) ne représentant pas plus du cinquième de celle de l'acide arachidonique (ARA) ;
b) on récolte *Pythium insidiosum ;*
c) on extrait l'huile du *Pythium insidiosum* récolté ; et
d) on recueille l'huile.

10. Procédé suivant la revendication 9, dans lequel les acides gras présents dans l'huile comprennent de 10 à 50 % d'ARA.

11. Procédé suivant la revendication 10, dans lequel les acides gras présents dans l'huile comprennent de 30 à 50 % d'ARA.

12. Procédé suivant l'une quelconque des revendications 9 à 11, dans lequel l'huile comprend au moins 10 parties d'ARA par partie d'EPA.

13. Procédé suivant la revendication 12, dans lequel l'huile comprend au moins 20 parties d'ARA par partie d'EPA.

14. Procédé suivant l'une quelconque des revendications 9 à13, dans lequel on maintient le niveau de l'oxygène dissous dans le milieu de culture entre 10 % et 50 % de la valeur de saturation en air du milieu pendant toute la culture.

15. Procédé suivant l'une quelconque des revendications 9 à14, dans lequel la source de carbone est du glucose apporté en une concentration de 10 à 100 g de glucose par litre de milieu et la source d'azote est un extrait de levure apporté en une concentration de 2 à 15 g d'extrait de levure par litre de milieu.

16. ARA raffiné contenant de l'huile fongique, dans lequel les acides gras présents dans l'huile fongique comprennent de 30 à 50 % d'acide arachidonique (ARA) et une quantité d'acide eicosapentaènoïque (EPA) ne représentant pas plus du cinquième de la quantité d'acide arachidonique (ARA) et dans lequel l'huile fongique est choisie dans le groupe consistant en des huiles fongiques produites par *Pythium insidiosum,* l'huile fongique étant appropriée à une utilisation dans la préparation d'une composition telle que revendiquée dans l'une quelconque des revendications 2 à 8.
